# EUROPEAN PATENT APPLICATION

(11) **EP 4 349 340 A1**
(43) Date of publication of application: **10.04.2024**
(21) Application number: 22815267.4
(22) Date of filing: 31.05.2022
(51) Int. Cl.: A61K 31/506, A61K 31/519, A61K 31/496, A61K 31/44, A61P 35/00

(54) **USE OF PHARMACEUTICAL COMPOSITION FOR TREATING LUNG CANCER**

(30) Priority: 04.06.2021 US 202163196756 P
(71) Applicant: Hung, Mien-Chie, Taichung City 404, Taiwan (TW)
(72) Inventor: TSAI, Chang-hai, Taichung City 408 Taiwan (CN); HUNG, Mien-chie, Taichung City 404 Taiwan (CN); LEE, Pei-chih, Taichung City 404 Taiwan (CN); HSIAO, Yu-chun, Taipei City 241 Taiwan (CN); TSENG, Tzu-yu, Taichung City 411 Taiwan (CN)
(74) Representative: D Young & Co LLP
(86) International application number: PCT/CN2022/096233
(87) International publication number: WO 2022/253222

(57) **Abstract**

Disclosed is the use of a pharmaceutical composition for treating lung cancer. The pharmaceutical composition comprises a nucleoprotein kinase C-δ inhibitor, and the nucleoprotein kinase C-δ inhibitor is abemaciclib, albendazole, amoxanpine, ceritinib dihydrochloride, cobimetinib, dabigatran etexilate, dabrafenib, emamectin, ingenol, ixazomib, midostaurin, niclosamide, nintedanib ethanesulfonate salt, ponatinib, raloxifene, regorafenib, ruxolitinib, tafenoquine, travoprost, mitochondrial antioxidant, vortioxetine, or derivatives thereof. Therefore, the pharmaceutical composition can be used for preparing drugs for treating lung cancer.

## Description

### Technical Field

The present disclosure relates to a use of a pharmaceutical composition. More particularly, the present disclosure relates to a use of a pharmaceutical composition including nuclear protein kinase C-δ inhibitor for treating lung cancer.

### Description of Related Art

Lung cancer has always been the most prevalent and lethal cancer. Among the global cancer cases, approximately 20% thereof are patients suffered from lung cancer, and the five-year survival rate among the patients is only 15%. In histopathology, lung cancer can be broadly categorized into non-small-cell lung cancer and small-cell lung cancer, wherein about 85%-90% of lung cancer cases belong to the non-small-cell lung cancer. Depending on the different degrees of invasion by the non-small-cell lung cancer, different treatments can be adopted. For the non-small-cell lung cancer in an early stage (Stage I and Stage II), a surgery can be adopted to fully remove the tumor. For the patients in advanced stages (Stage III and Stage IV) or the patients not able to endure surgery, a treatment of chemotherapy and radiotherapy can be adopted to achieve better therapeutic outcomes.

Furthermore, targeted therapy is another option for the treatment of the non-small-cell lung cancer. A typical drug for clinical targeted therapy is epidermal growth factor receptor-tyrosine kinase inhibitors (EGFR-TKIs), which can bind to the epidermal growth factor receptor (EGFR) and disrupt signal transduction, thereby achieving the effect of inhibiting the proliferation and metastasis of cancer cells. However, in clinical practice, around 50% of non-small-cell lung cancer patients have EGFR gene mutations, resulting in an overexpression of EGFR on the cancer cells thereof. Even be treated with EGFR-TKIs, there is a possible recurrence within two years after the treatment. Also, the patients will develop resistance to EGFR-TKIs, and no other proper drug can be used for further treatment after recurrence.

In this regard, the development of a drug for the treatment of lung cancer with EGFR mutations and resistance to EGFR-TKIs is still pursued by the researchers and the industry.

### SUMMARY

In order to achieve the aforementioned goal, the purpose of the present disclosure is to provide a pharmaceutical composition for treating lung cancer, which can be used in combination with EGFR-TKIs to treat the lung cancer with EGFR mutations and resistance to EGFR-TKIs, so as to extend the usage of EGFR-TKIs.

According to one aspect of the present disclosure, a use of a pharmaceutical composition is to prepare a drug for treating lung cancer. The pharmaceutical composition includes a nuclear protein kinase C-δ inhibitor. The nuclear protein kinase C-δ inhibitor includes abemaciclib, albendazole, amoxapine, ceritinib dihydrochloride, cobimetinib, dabigatran etexilate, dabrafenib, emamectin, ingenol, ixazomib, midostaurin, niclosamide, nintedanib esylate, ponatinib, raloxifene, regorafenib, ruxolitinib, tafenoquine, travoprost, visomitin, vortioxetine or derivatives thereof.

Therefore, the pharmaceutical composition of the present disclosure can reduce the resistance of lung cancer cells to EGFR-TKIs by using the nuclear protein kinase C-δ inhibitor. The pharmaceutical composition is suitable for preparing the drug for treating lung cancer, especially the drug for treating lung cancer with EGFR mutations and resistance to EGFR-TKIs.

According to the aforementioned use of the pharmaceutical composition, wherein a concentration of the nuclear protein kinase C-δ inhibitor can be 0.05 µM to 10 µM.

According to the aforementioned use of the pharmaceutical composition, wherein the pharmaceutical composition can further include epidermal growth factor receptor-tyrosine kinase inhibitors.

According to the aforementioned use of the pharmaceutical composition, wherein a concentration of the epidermal growth factor receptor-tyrosine kinase inhibitors can be 0.05 µM to 1 µM.

According to the aforementioned use of the pharmaceutical composition, wherein the epidermal growth factor receptor-tyrosine kinase inhibitors can be gefitinib.

According to the aforementioned use of the pharmaceutical composition, wherein the nuclear protein kinase C-δ inhibitor can be nintedanib esylate, a concentration of nintedanib esylate can be 0.5 µM to 5 µM, and a concentration of gefitinib can be 0.05 µM to 1 µM.

According to the aforementioned use of the pharmaceutical composition, wherein the nuclear protein kinase C-δ inhibitor can be ponatinib, a concentration of ponatinib can be 0.05 µM to 0.5 µM, and a concentration of gefitinib can be 0.05 µM to 1 µM.

According to the aforementioned use of the pharmaceutical composition, wherein the nuclear protein kinase C-δ inhibitor can be regorafenib, a concentration of regorafenib can be 1 µM to 10 µM, and a concentration of gefitinib can be 0.05 µM to 1 µM.

According to the aforementioned use of the pharmaceutical composition, wherein the nuclear protein kinase C-δ inhibitor can be cobimetinib, a concentration of cobimetinib can be 1 µM to 10 µM, and a concentration of gefitinib can be 0.05 µM to 1 µM.

According to the aforementioned use of the pharmaceutical composition, wherein the lung cancer can be non-small-cell lung cancer.

According to the aforementioned use of the pharmaceutical composition, wherein the lung cancer can exhibit resistance to the epidermal growth factor receptor-tyrosine kinase inhibitors.

### BRIEF DESCRIPTION OF THE DRAWINGS

In order to make the above and other objects, features, advantages and examples of the present disclosure more obvious and understandable, the accompanying drawings are described as follows:
Fig. 1 shows a confocal-based high-content screening system that measures the quantity of nuclear protein kinase C-δ;
Fig. 2A, Fig. 2B, Fig. 2C, Fig. 2D, Fig. 2E and Fig. 2F respectively show immunofluorescence staining images of the GR10 cells after treatment of Example 1, Example 2, Example 3, Example 4, Comparative Example 1 and Comparative Example 2;
Fig. 3 is a comparative diagram of fluorescence intensity of the GR10 cells after treatment of Example 1 to Example 4, Comparative Example 1 and Comparative Example 2;
Fig. 4A, Fig. 4B, Fig. 4C and Fig. 4D respectively show the relationship diagrams between cell viabilities and concentrations for the GR6 cells after treatment of Example 5, Example 6, Example 7 and Example 8; and
Fig. 5A, Fig. 5B, Fig. 5C and Fig. 5D respectively show the relationship diagrams between cell viabilities and concentrations for the GR10 cells after treatment of Example 9, Example 10, Example 11 and Example 12.

### DETAILED DESCRIPTION

According to the present disclosure, a use of a pharmaceutical composition is to prepare a drug for treating lung cancer. The pharmaceutical composition includes a nuclear protein kinase C-δ (nPKCδ) inhibitor. The nuclear protein kinase C-δ inhibitor includes abemaciclib, albendazole, amoxapine, ceritinibdihydrochloride, cobimetinib, dabigatran etexilate, dabrafenib, emamectin, ingenol, ixazomib, midostaurin, niclosamide, nintedanibesylate, ponatinib, raloxifene, regorafenib, ruxolitinib, tafenoquine, travoprost, visomitin, vortioxetine or derivatives thereof.

According to the pharmaceutical composition of the present disclosure, the concentration of the nuclear protein kinase C-δ inhibitor can be 0.05 µM to 10 µM. The pharmaceutical composition of the present disclosure can further include epidermal growth factor receptor-tyrosine kinase inhibitors (EGFR-TKIs). The concentration of the epidermal growth factor receptor-tyrosine kinase inhibitors can be 0.05 µM to 1 µM, and the epidermal growth factor receptor-tyrosine kinase inhibitors can be gefitinib.

It should be noticed that, the concentrations of the nuclear protein kinase C-δ inhibitor and the epidermal growth factor receptor-tyrosine kinase inhibitors can be adjusted based on the types thereof.

For example, when the nuclear protein kinase C-δ inhibitor is nintedanib esylate, the concentration of nintedanib esylate can be 0.5 µM to 5 µM, and the concentration of gefitinib can be 0.05 µM to 1 µM. When the nuclear protein kinase C-δ inhibitor is ponatinib, the concentration of ponatinib can be 0.05 µM to 0.5 µM, and the concentration of gefitinib can be 0.05 µM to 1 µM. When the nuclear protein kinase C-δ inhibitor is regorafenib, the concentration of regorafenib can be 1 µM to 10 µM, and the concentration of gefitinib can be 0.05 µM to 1 µM. When the nuclear protein kinase C-δ inhibitor is cobimetinib, the concentration of cobimetinib can be 1 µM to 10 µM, and the concentration of gefitinib can be 0.05 µM to 1 µM.

The lung cancer targeted for treatment with the pharmaceutical composition of the present disclosure can be non-small-cell lung cancer. Furthermore, the lung cancer targeted for treatment with the pharmaceutical composition of the present disclosure can exhibit resistance to the epidermal growth factor receptor-tyrosine kinase inhibitors.

Unless otherwise noted, all terms, symbols or other scientific terms or terms used in the present disclosure have the meanings that are commonly understood by person having ordinary skill in the art. In some cases, terms with conventional meanings are defined herein for clarity and/or immediate reference, and the definitions incorporated herein should be construed as not necessarily substantial different from the conventional meanings in the art. Many of the techniques and procedures described or referenced herein are well known and routinely used by those skilled in the art. Where appropriate, unless otherwise stated, procedures for the use of commercially available kits and reagents are generally performed according to instructions and/or parameters defined by the manufacturer.

The nuclear protein kinase C-δ inhibitor disclosed in the present disclosure can be verified through in vitro tests to inhibit the growth of non-small-cell lung cancer cells resistant to the epidermal growth factor receptor-tyrosine kinase inhibitors. Furthermore, it can be verified through in vivo tests that the nuclear protein kinase C-δ inhibitor disclosed in the present disclosure can be administered to animals (such as mouse models) with lung cancer resistant to the epidermal growth factor receptor-tyrosine kinase inhibitors, and the therapeutic effects can be obtained. Positive results in one or more tests is sufficient to demonstrate the actual utility of the tested inhibitor, and an appropriate dosage range and administration route for animals (such as humans) can be determined based on test results.

Useful pharmaceutical dosage forms for administering the nuclear protein kinase C-δ inhibitor of the present disclosure include, but are not limited to, hard and soft gelatin capsules, tablets, parenteral injections and oral suspensions. The dosage administered can depend on factors including the age of the subject, the health and weight of the subject, the extent of the disease, the type of concomitant treatment (if any), the frequency of treatment and the nature of the desired effect. Usually the daily dose of active ingredient may vary, for example from 0.1 to 2000 mg per day, or 10-500 mg one or more times per day may be effective to achieve the desired results.

The same dosage form can generally be used when the nuclear protein kinase C-δ inhibitor of the present disclosure are administered stepwise or in combination with at least one other therapeutic agent. When drugs are administered in a physical combination, the dosage form and route of administration should be selected based on the compatibility of the combined drugs. Therefore, "co-administration" in the specification should be understood to include the concomitant or sequential administration of at least two agents, or as a fixed-dose combination of at least two active ingredients.

The nuclear protein kinase C-δ inhibitor of the present disclosure can be used as the active ingredient alone, or administered in combination with at least one second active ingredient, the second active ingredient can be selected from, for example, other active ingredients known to be useful in the treatment of patients with the non-small-cell lung cancer, in particular the epidermal growth factor receptor-tyrosine kinase inhibitors.

The present disclosure will be further exemplified by the following specific embodiments so as to facilitate utilizing and practicing the present disclosure completely by the people skilled in the art without over-interpreting and over-experimenting. However, the readers should understand that the present disclosure should not be limited to these practical details thereof, that is, these practical details are used to describe how to implement the materials and methods of the present disclosure and are not necessary.

### <Inhibitory Effect of Nuclear Protein Kinase C-δ Inhibitor on Non-Small-Cell Lung Cancer Cells Resistant to Epidermal Growth Factor Receptor-Tyrosine Kinase Inhibitors>

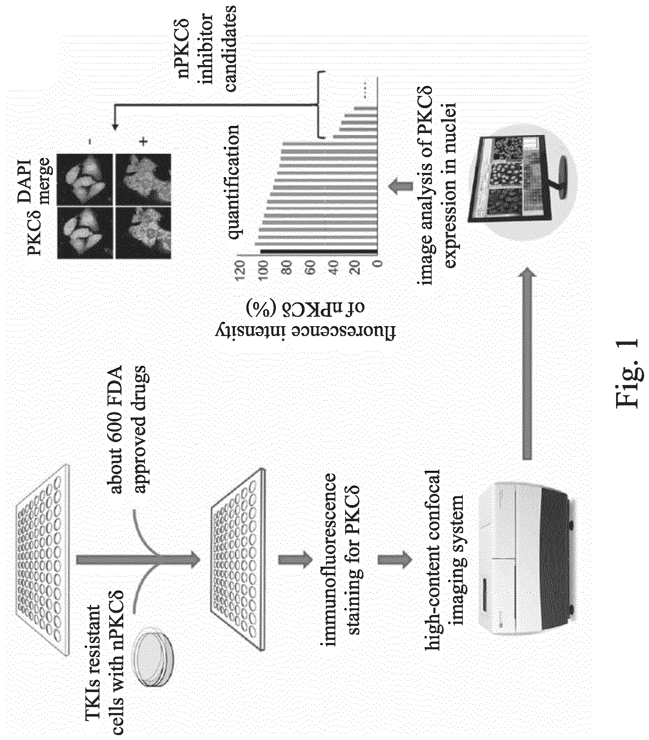

Please refer to Fig. 1. Fig. 1 shows a confocal-based high-content screening system that measures the quantity of nuclear protein kinase C-δ. To investigate the growth-inhibiting effect of the nuclear protein kinase C-δ inhibitor of the present disclosure on the non-small-cell lung cancer cells resistant to the epidermal growth factor receptor-tyrosine kinase inhibitors, the present experimental example utilizes the screening system shown in Fig. 1 to measure whether the nuclear protein kinase C-δ inhibitor of the present disclosure can inhibit the production of nuclear protein kinase C-δ in the cancer cells.

In the present experimental example, gefitinib-resistant non-small-cell lung cancer cells (gefitinib-resistant 10 cells, hereinafter "GR10 cells") are used as the cell model. In Example 1 to Example 4, GR10 cells are respectively treated with nintedanib esylate (hereinafter "Nin"), ponatinib (hereinafter "Pon"), regorafenib (hereinafter "Reg") or cobimetinib (hereinafter "Cob") for 24 hours, and the quantity of nuclear protein kinase C-δ within the GR10 cells is determined by confocal-based high-content image analysis. On the other hand, the present experimental example further includes Comparative Example 1 and Comparative Example 2. GR10 cells are untreated in Comparative Example 1, and GR10 cells are treated with non-medical sotrastaurin (hereinafter "Sotra") in Comparative Example 2.

The types and concentrations of the nuclear protein kinase C-δ inhibitors used in Example 1 to Example 4, Comparative Example 1 and Comparative Example 2 are shown in the following Table 1.

| Table 1 | | | | | | |
|---|---|---|---|---|---|---|
| | Example 1 | Example 2 | Example 3 | Example 4 | Comparative Example 1 | Comparative Example 2 |
| Type of Inhibitor | Nin | Pon | Reg | Cob | None | Sotra |
| Concentration of Inhibitor (µM) | 5-10 | 0.1-0.5 | 5-10 | 5-10 | N/A | 5-10 |

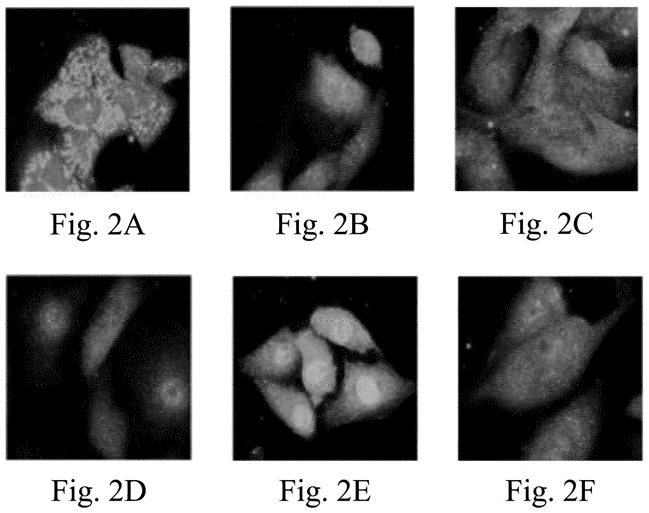

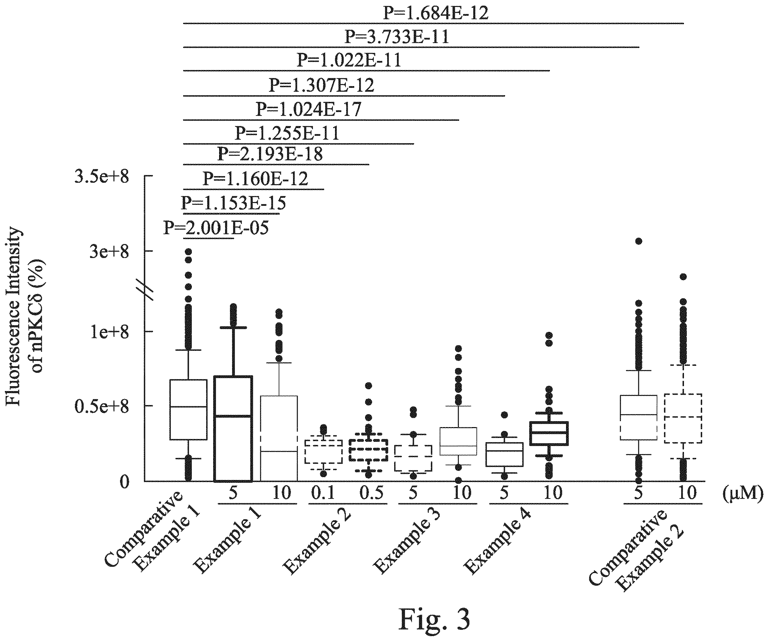

Please refer to Fig. 2A to Fig. 2F and Fig. 3. Fig. 2A to Fig. 2F respectively show immunofluorescence staining images of the GR10 cells after treatment of Example 1, Example 2, Example 3, Example 4, Comparative Example 1 and Comparative Example 2. Fig. 3 is a comparative diagram of fluorescence intensity of the GR10 cells after treatment of Example 1 to Example 4, Comparative Example 1 and Comparative Example 2. In Fig. 2A to Fig. 2F, the concentration of nuclear protein kinase C-δ is higher when the color of cells is lighter (closer to white).

From Fig. 2A to Fig. 2F, it can be observed that, compared to the untreated GR10 cells (Comparative Example 1), the nuclear protein kinase C-δ inhibitor of the present disclosure can exert inhibitory effects on nuclear protein kinase C-δ of the GR10 cells, and the inhibitory effects thereof are not less than the inhibitory effect of the non-medical sotrastaurin (Comparative Example 2). From Fig. 3, it can be understood that, when the concentration of Nin is greater than 5 µM, Pon is greater than 0.1 µM, Reg is greater than 5 µM or Cob is greater than 5 µM, a great ability to inhibit nuclear protein kinase C-δ can be performed.

### <Synergistic Inhibition of Growth of Non-Small-Cell Lung Cancer Cells Resistant to Epidermal Growth Factor Receptor-Tyrosine Kinase Inhibitors by Nuclear Protein Kinase C-δ Inhibitor in Combination with Epidermal Growth Factor Receptor-Tyrosine Kinase Inhibitors>

In the present experimental example, the GR10 cells used in the previous experimental example and GR6 cells are chosen for the following experiments. In summary, the present experimental example includes Example 5 to Example 12, where different concentrations of the nuclear protein kinase C-δ inhibitor (Nin, Pon, Reg or Cob) are used in combination with gefitinib (hereinafter "Gef") to treat the GR6 cells or the GR10 cells. After 3 days of treatment, the cell viability of each example is determined. The types of cells, types and concentrations of the nuclear protein kinase C-δ inhibitor and Gef concentrations used in Example 5 to Example 12 are listed in Table 2 and Table 3 as follows.

| Table 2 | | | | |
|---|---|---|---|---|
| | Example 5 | Example 6 | Example 7 | Example 8 |
| Type of Cells | GR6 Cells | | | |
| Type of Inhibitor | Nin | Pon | Reg | Cob |
| Concentration of Inhibitor (µM) | 0.5-4 | 0.05-0.4 | 1-8 | 4-8 |
| Concentration of Gef (µM) | 0.05-1 | 0.05-1 | 0.1-0.8 | 0.1-0.8 |

| Table 3 | | | | |
|---|---|---|---|---|
| | Example 9 | Example 10 | Example 11 | Example 12 |
| Type of Cells | GR10 Cells | | | |
| Type of Inhibitor | Nin | Pon | Reg | Cob |
| Concentration of Inhibitor (µM) | 0.5-4 | 0.05-0.4 | 1-8 | 4-8 |
| Concentration of Gef (µM) | 0.05-1 | 0.05-1 | 0.1-0.8 | 0.1-0.8 |

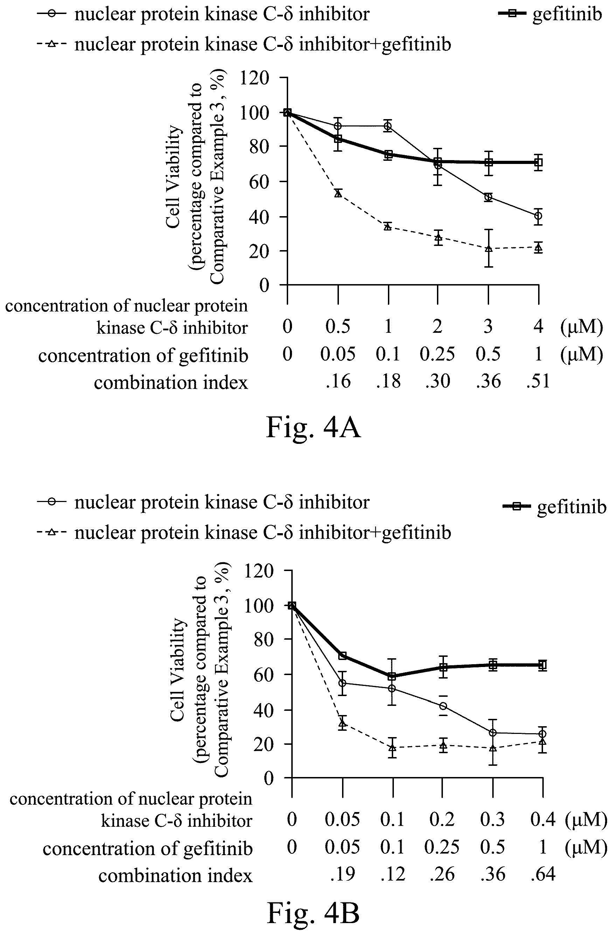

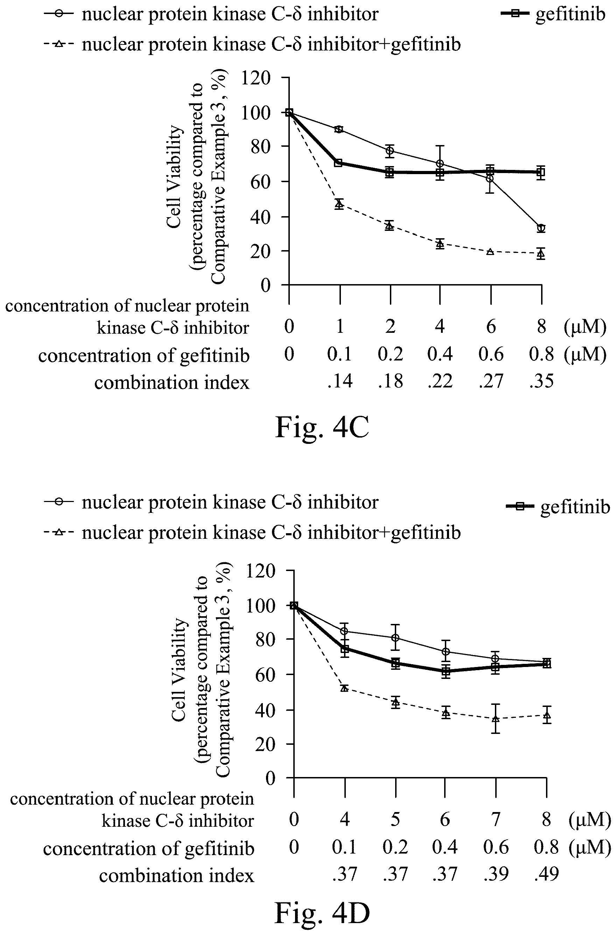

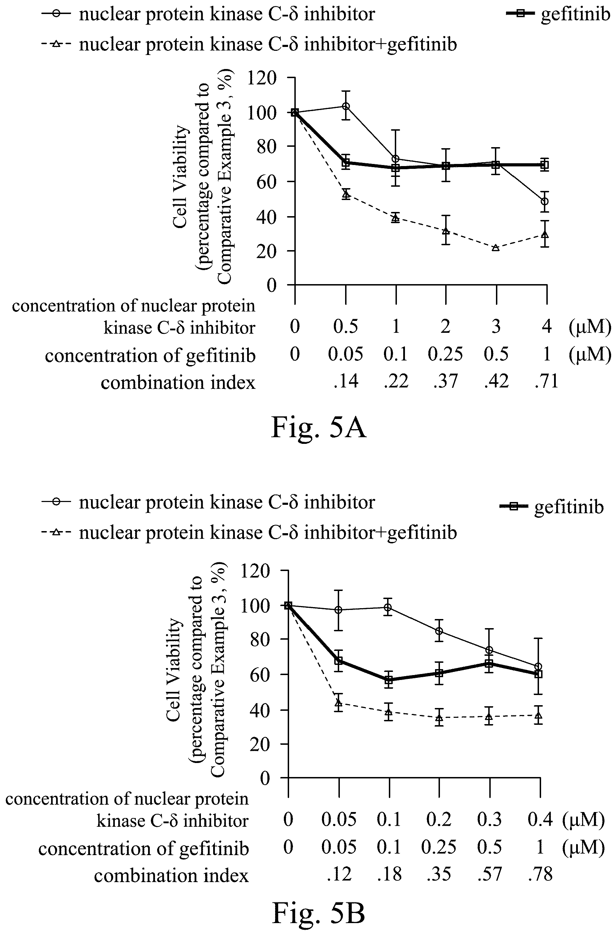

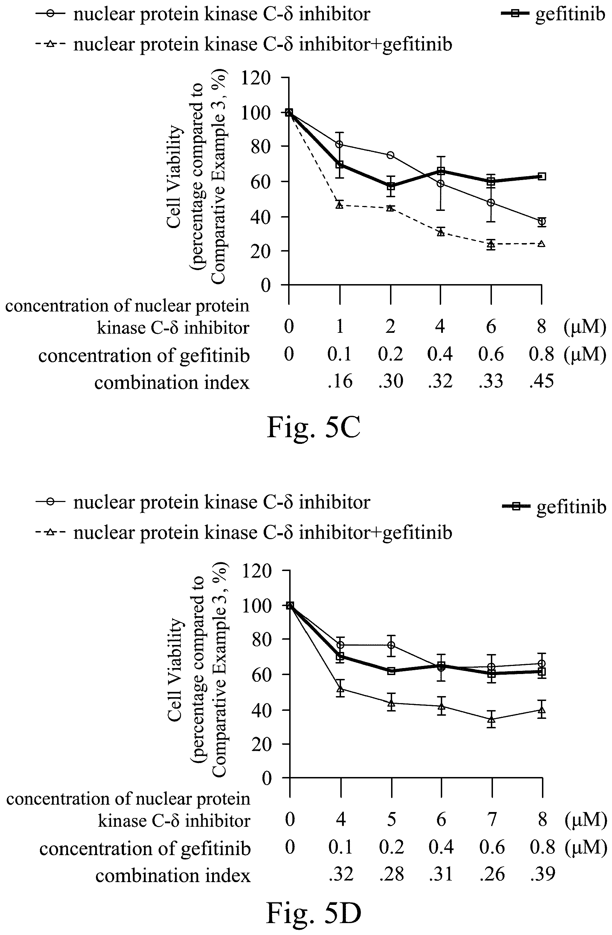

Please refer to Fig. 4A to Fig. 4D and Fig. 5A to Fig. 5D. Fig. 4A to Fig. 4D respectively show the relationship diagrams between cell viabilities and concentrations for the GR6 cells after treatment of Example 5, Example 6, Example 7 and Example 8. Fig. 5A to Fig. 5D respectively show the relationship diagrams between cell viabilities and concentrations for the GR10 cells after treatment of Example 9, Example 10, Example 11 and Example 12. The aforementioned cell viabilities are obtained through the MTT tests, and the combination index (CI) of nuclear protein kinase C-δ and Gef of each example is calculated to understand whether nuclear protein kinase C-δ and Gef can perform synergy.

From Fig. 4A to Fig. 4D and Fig. 5A to Fig. 5D, it can be understood that the nuclear protein kinase C-δ inhibitor of the present disclosure can reduce the cell viabilities of both the GR6 cells and the GR10 cells. After adding Gef, the cell viabilities of the GR6 cells and the GR10 cells are significantly decreased, and nuclear protein kinase C-δ and Gef are able to perform obvious synergy (CI < 1). Furthermore, at specific concentrations, nuclear protein kinase C-δ and Gef are able to perform strong synergy (CI < 0.3), further reducing the cell viabilities of the GR6 cells and the GR10 cells.

In this regard, the pharmaceutical composition of the present disclosure can reduce the resistance of lung cancer cells to EGFR-TKIs by using the nuclear protein kinase C-δ inhibitor. The pharmaceutical composition is suitable for preparing the drug for treating lung cancer, especially the drug for treating lung cancer with EGFR mutations and resistance to EGFR-TKIs.

Although the present disclosure has been described in considerable detail with reference to certain embodiments thereof, other embodiments are possible. Therefore, the spirit and scope of the appended claims should not be limited to the description of the embodiments contained herein.

## Claims

1. A use of a pharmaceutical composition, which is to prepare a drug for treating lung cancer, **characterized in that**, the pharmaceutical composition comprising a nuclear protein kinase C-δ (nPKCδ) inhibitor;
wherein the nuclear protein kinase C-δ inhibitor comprises abemaciclib, albendazole, amoxapine, ceritinibdihydrochloride, cobimetinib, dabigatran etexilate, dabrafenib, emamectin, ingenol, ixazomib, midostaurin, niclosamide, nintedanibesylate, ponatinib, raloxifene, regorafenib, ruxolitinib, tafenoquine, travoprost, visomitin, vortioxetine or derivatives thereof.

2. The use of the pharmaceutical composition of claim 1, **characterized in that**, a concentration of the nuclear protein kinase C-δ inhibitor is 0.05 µM to 10 µM.

3. The use of the pharmaceutical composition of claim 1, **characterized in that**, the pharmaceutical composition further comprises epidermal growth factor receptor-tyrosine kinase inhibitors (EGFR-TKIs).

4. The use of the pharmaceutical composition of claim 3, **characterized in that**, a concentration of the epidermal growth factor receptor-tyrosine kinase inhibitors is 0.05 µM to 1 µM.

5. The use of the pharmaceutical composition of claim 3, **characterized in that**, the epidermal growth factor receptor-tyrosine kinase inhibitors is gefitinib.

6. The use of the pharmaceutical composition of claim 5, **characterized in that**, the nuclear protein kinase C-δ inhibitor is nintedanib esylate, a concentration of nintedanib esylate is 0.5 µM to 5 µM, and a concentration of gefitinib is 0.05 µM to 1 µM.

7. The use of the pharmaceutical composition of claim 5, **characterized in that**, the nuclear protein kinase C-δ inhibitor is ponatinib, a concentration of ponatinib is 0.05 µM to 0.5 µM, and a concentration of gefitinib is 0.05 µM to 1 µM.

8. The use of the pharmaceutical composition of claim 5, **characterized in that**, the nuclear protein kinase C-δ inhibitor is regorafenib, a concentration of regorafenib is 1 µM to 10 µM, and a concentration of gefitinib is 0.05 µM to 1 µM.

9. The use of the pharmaceutical composition of claim 5, **characterized in that**, the nuclear protein kinase C-δ inhibitor is cobimetinib, a concentration of cobimetinib is 1 µM to 10 µM, and a concentration of gefitinib is 0.05 µM to 1 µM.

10. The use of the pharmaceutical composition of claim 1, **characterized in that**, the lung cancer is non-small-cell lung cancer.

11. The use of the pharmaceutical composition of claim 1, **characterized in that**, the lung cancer exhibits resistance to epidermal growth factor receptor-tyrosine kinase inhibitors.
